# EUROPEAN PATENT SPECIFICATION

(11) **EP 3 582 744 B1**
(45) Date of publication and mention of the grant of the patent: **24.05.2023**
(21) Application number: 17719902.3
(22) Date of filing: 16.02.2017
(51) Int. Cl.: A61J 15/00, A61M 25/04

(54) **GASTROSTOMY DEVICE WITH AN IMPROVED RETAINING ELEMENT**
GASTROSTOMIEVORRICHTUNG MIT VERBESSERTEM HALTEELEMENT
DISPOSITIF DE GASTROSTOMIE POURVU D'UN ÉLÉMENT DE RETENUE AMÉLIORÉ

(43) Date of publication of application: 25.12.2019
(73) Proprietor: N.V. Nutricia, 2712 HM Zoetermeer (NL); BS Medical Tech Industry Sarl, 67470 Niederroedern (FR)
(72) Inventor: WOLKENSTOERFER, Reinhold, 91077 Neunkirchen (DE); BASCH, Bertrand, 67620 Soufflenheim (FR)
(74) Representative: Schornack, Oliver
(86) International application number: PCT/IB2017/000351
(87) International publication number: WO 2018/150219

(56) References cited:
- DE-U1- 20 303 982
- US-A- 5 447 497
- US-A1- 2010 318 094
- US-A1- 2014 336 585
- US-B1- 8 177 742

## Description

The present invention relates to a gastrostomy device, and in particular to a gastrostomy device for percutaneous endoscopic gastrostomy (PEG), which has a double-layered retaining element for holding the gastrostomy device in place.

Percutaneous endoscopic gastrostomy is an endoscopic medical procedure in which a tube (PEG tube) is passed into a patient's stomach through the abdominal wall. Typically, such PEG tube serves to provide a means of feeding when oral intake is not adequate or possible. The procedure is an alternative to surgical gastrostomy insertion, and does not require a general anesthetic. PEG tubes may also be extended into the small intestine by passing a jejunal extension tube (PEG-J tube) through the PEG tube and into the jejunum via the pylorus.

In order to place the PEG tube, the abdominal wall and the stomach is punctured with a trocar. A distal end of the PEG tube is then inserted into the stomach. A balloon at the distal end of the PEG tube and an external retainer slidably arranged on the PEG tube hold the PEG tube in place and pull the stomach wall and abdominal wall together. Such balloon as well as the PEG tube are usually made of a silicone material, which allows flexibility for the PEG tube and due to the prolongation of the silicone material an inflatable and self-deflating balloon.

For instance, US 8,177,742 B1 relates to an inflatable retention system for an enteral feeding device including an inflatable balloon having a predetermined full volume. The balloon may be made of PU, PVC or PA or other materials as well as admixtures or blends of these materials. The inflatable balloon may stretch or distend, e.g. about 10% to 15% deformation depending on the pressure of the inflated balloon.

On the other hand, US 2010/0318094 A1 relates to a guide tube equipped with a balloon for puncture, wherein the tube is inserted into the esophagus to form a cervical esophageal fistula. The balloon is punctured with a puncture needle. The balloon is formed of a synthetic resin, such as a soft vinyl chloride resin, a polyurethane resin, a silicon rubber, polyethylene, polyester, or natural rubber latex.

However, the balloon of a PEG tube is subject to mechanical influences due to movement of the stomach or the torso of the patient and is also subject to influences of the stomach fluids. Since a PEG tube has to stay in place for approximately two to four weeks (or sometimes longer) until a stoma is formed and healed, it is dangerous and disadvantageous if the balloon breaks or otherwise loses internal pressure.

It is therefore an object of the present invention to provide a gastrostomy device that is durable and provides improved retaining capabilities.

This object is solved by the present invention as defined in the independent claims. Preferred embodiments are defined by the dependent claims.

In accordance with an aspect of the present disclosure, a gastrostomy device comprises a gastrostomy tube and an inflatable retaining element coupled to the tube at or near a distal end of the tube. The retaining element comprises an inner layer made of polyurethane, PUR (or PU), and an outer layer made of polyamide, PA.

PUR and PA allow forming an inflatable retaining element in any desired form, since PA provides form stability and durability even over a longer period of time. Thus, an inflated retaining element made of or with PA maintains its form and has a small mechanical prolongation in spite of any internal pressure when inflated. Moreover, PUR provides softness to the retaining element preventing the stomach wall from irritations or injury, and in particular significantly reduces the risk of necrosis. The retaining element can be fabricated from any suitable PUR and PA material. The following lists include some examples:

| Inner layer | Material name | Shore hardness DIN ISO 7619-1 (3s) / ISO 868 | g/cm³ |
|---|---|---|---|
| | Elastollan^{®} 1170A 10 | 71A | 1,08 |
| | Elastollan^{®} 1175A 10 W | 75A | 1,14 |
| | Elastollan^{®} 1180A 10 | 80A | 1,11 |
| | Elastollan^{®} 1185A 10 W | 83A | 1,16 |
| | Elastollan^{®} 1185A 10 | 87A / 36D | 1,12 |
| | Elastollan^{®} 1185A 59 U | 86A | 1,12 |
| | Elastollan^{®} 1185A 10 M | 88A / 39D | 1,11 |
| | Elastollan^{®} 1185A 12 WM | 87A / 39D | 1,13 |
| | Elastollan^{®} 1190A 10 | 92A / 42D | 1,13 |
| | Elastollan^{®} 1195A 10 | 96A / 48D | 1,15 |
| | Elastollan^{®} 1195 A 55 U | 43D | 1,15 |
| | Elastollan^{®} 1198A 10 | 52D | 1,17 |
| | Elastollan^{®} 1154D 10 | 53D | 1,17 |
| | Elastollan^{®} 1160D 50 | 60D | 1,18 |
| | Elastollan^{®} 1164D 11 | 69D | 1,18 |
| | Elastollan^{®} 1174D 11 | 75D | 1,2 |
| | Pellethane^{®} 2363-55DE | 53D | |
| | Pellethane^{®} 2363-90AE | 90A | |
| | Carbothane^{®} PC-3575A | 70A | |
| | Carbothane^{®} PC-3585A | 80A | |
| | Carbothane^{®} PC-3595A | 90A | |
| | Carbothane^{®} PC-3555D | 50D | |
| | Carbothane^{®} PC-3572D | 69D | |
| | Carbothane^{®} PC-3575A-B20 | 75A | |
| | Carbothane^{®} PC-3585A-B20 | 80A | |
| | Carbothane^{®} PC-3595A-B20 | 92A | |
| | Carbothane^{®} PC-3555D-B20 | 52D | |
| | Carbothane^{®} PC-3572D-B20 | 71D | |
| | Carbothane^{®} AC-4075A | 77A | |
| | Carbothane^{®} AC-4085A | 85A | |
| | Carbothane^{®} AC-4095A | 95A | |
| | Carbothane^{®} AC-4055D | 56D | |
| | Carbothane^{®} AC-4075A-B20 | 78A | |
| | Carbothane^{®} AC-4085A-B20 | 90A | |
| | Carbothane^{®} AC-4095A-B20 | 95A | |
| | Carbothane^{®} AC-4055D-B20 | 55D | |
| | Quadraflex^{™} ARE-85 A | | |
| | Tecothane^{™} | | |
| | Estane^{®} | | |

| Outer layer | Name | Shore hardness DIN ISO 7619-1 (3s) / ISO 868 | g/cm³ |
|---|---|---|---|
| | Vestamid^{®} Care ME40 | 40D | 1,01 |
| | Vestamid^{®} Care ME47 | 47D | 1,03 |
| | Vestamid^{®} Care ME55 | 55D | 1,03 |
| | Vestamid^{®} Care ME62 | 62D | 1,03 |
| | Vestamid^{®} Care ME71 | 71D | 1,01 |
| | Vestamid^{®} Care ME40-B | 46D | 1,00 |
| | Vestamid^{®} Care ME55-B | 56D | 1,01 |
| | Vestamid^{®} Care ME62-B | 64D | 1,02 |
| | Vestamid^{®} Care ME71-B | 71D | 1,01 |
| | Pebax^{®} 2533 SA 01 MED | 27D | |
| | Pebax^{®} 3533 SA 01 MED | 33D | |
| | Pebax^{®} 4033 SA 01 MED | 42D | |
| | Pebax^{®} 4533 SA 01 MED | 46D | |
| | Pebax^{®} 5533 SA MED | 54D | |
| | Pebax^{®} 6333 SA MED | 64D | |
| | Pebax^{®} 7033 SA MED | 69D | |
| | Pebax^{®} 7233 SA MED | 69D | |
| | Pebax^{®} MV1074 SA01 MED | 40D | |
| | Rilsan^{®} MED PA11 | | |
| | Rilsan^{®} Clear MED Long Chain PA | | |
| | Rilsan^{®} MED PA12 | | |
| | Orgalloy PA 6... | | |
| | Grilamid L25 | 70D | 1,01 |

A preferable material for the inner layer is "Elastollan^{®} 1198A 10". For the outer layer a preferable material is "Vestamid^{®} Care ME62" being a polyether block amide, which is a thermoplastic elastomer made of flexible polyether and rigid polyamide. Both materials are preferably transparent and clear, but alternatively can be at least partially opaque and/or colored.

The inflatable retaining element can be formed by co-extrusion of PUR and PA, so that a double-layered tube is fabricated, where the PUR forms an inner layer and the PA forms an outer layer. Such double-layered tube is then widened at a certain portion thereof, for example, by inflating the certain portion of the tube into a mold. Before widening the double-layer tube it is advantageous to warm the double-layer tube, so that the PUR and PA layers can expand as uniformly as possible.

Optionally, the double-layered tube can be elongated or stretch formed before widening. By elongating or stretch forming the double-layered tube a diameter thereof can be adapted (reduced), so that the double-layered tube fits onto the tube of the gastrostomy device, i.e. an inner diameter of the double-layered tube corresponds to an outer diameter of the tube of the gastrostomy device.

The mold is provided with an inner surface having the form of the resulting retaining element. For instance, the mold consists of one or more parts. In case of multiple parts the double-layered tube can be put into a first part and one or more further parts are closed over the double-layered tube to form the complete mold. In order to avoid defects at the resulting retaining element, the mold can be warmed, thereby avoiding instant curing or hardening of the double-layered tube during widening. After curing (e.g. cooling down) the retaining element can be taken out of the mold. Any remaining parts of the double-layered tube at one or both ends of the retaining element can be cut to a desired length.

Alternatively or additionally, the inner layer can be formed by extrusion or spraying raw material onto a mold. Also alternatively or additionally, the outer layer is then also extruded or sprayed onto the inner layer on the mold. Another example of forming the inner layer and applying the outer layer is dipping. For instance, a pre-formed retaining element consisting only of the inner layer can be dipped into a dip for forming the outer layer. After curing of the materials of the inner and outer layers, such as PUR and PA, the mold is removed leaving the finished retaining element. For example, the mold can be an inflatable mold or a dissolvable mold which is dissolved and flushed out of the retaining element.

The inner layer made of PUR of the tube may have a thickness of 0.1 mm to 0.3 mm, preferably 0.2 mm, and the outer layer made of PA may have a thickness of 0.05 mm to 0.2 mm, preferably 0.1 mm. After elongating or stretching the tube the combined thickness of the inner and outer layer may be reduced to 0.1 mm to 0.35 mm, but may be preferably 0.15 mm.

Furthermore, when forming the inflatable retaining element, e.g. by widening a certain portion of the tube, the thickness of the inner and/or outer layer will be reduced. Since the retaining element has increased dimensions compared to the tube (e.g. an increased diameter), the thickness of the inner and outer layer will approximately linearly decrease from a portion where the tube is not widened to a radially outer most portion of the widened tube. For instance, at the radially outer most portion the combined thickness of the inner and outer layers may be as small as approximately 0.025 mm. As an example only, the thickness of the inner layer may be reduced to 0.02 mm and the thickness of the outer layer may be reduced to 0.005 mm due to the widening. Such thin layers provide for an easy to fold retaining element that, when in an empty state, does not require much space. The overall thickness (diameter) of the distal end of the gastrostomy device, when the retaining element is in its empty state, therefore, can be kept small and avoids big sheathes for placing the gastrostomy device.

Due to the double layer structure of the retaining element the mechanical tear strength is increased compared to a retaining element made of only one layer, such as a single PUR layer. In addition, the double layer structure also prevents creep effects that would otherwise occur in a mono layer structure. A further advantage of PUR and PA is that the gastrostomy device can be made silicone free. A plurality of conventional gastrostomy tubes and catheters include a silicone to achieve the required flexibility. Particularly retaining elements, such as balloons, of gastrostomy devices were made of silicone to provide an inflatable balloon based on the prolongation of the silicone. However, a plurality of patients are allergic to silicone. Therefore, PUR and PA is more compatible with the majority of patients.

The outer layer made of PA increases the durability of the retaining element. Specifically, the mechanical prolongation of PA is less than that of PUR. Thus, when inflated, the retaining element will achieve the desired form and will maintain this form if mechanical influences from the stomach or movement of the torso of the patient are acting on the retaining element. In addition, the retaining element made of PUR and PA provides the advantage that in case of a small leakage it will maintain its form over a longer period of time and, hence, will retain the gastrostomy device in position for a longer period of time. Such retaining element made of PUR and PA has a small extensibility. For instance, an elastic elongation of the retaining element is considerably below 30%.

A conventional silicone balloon, on the other hand, can be squeezed (deformed) by external influences which may decrease a secure retaining of the gastrostomy device. In addition, due to the elasticity and very low tear strength of silicone a small hole or a mechanical damage like a scratch in a silicone balloon will lead to an immediate balloon burst and, therefore, a sudden pressure loss as the fluid within the balloon will be squeezed out very quickly. For instance, a silicone balloon may be elastically elongated easily above 500%. Thus, the silicone gastrostomy device may suddenly come loose or even move out of the stoma.

Furthermore, PA provides a high acid resistance, so that the stomach body fluid has less influence on the material of the retaining element. The outer PA layer also increases safety properties of the retaining element, since it significantly reduces leakage or damage of the double layer skin forming the retaining element. For instance, when fabricating the retaining element, compounds or gels of PUR and PA may not completely melt and can form pinholes. That two of such pinholes, one in the PUR layer and one in the PA layer, are exactly at the same position is almost impossible.

Furthermore, chemical properties of PA, such as hydrolysis, water and/or gas permeability and electrolyte migration, are better than those of PUR. Thus, there will be almost no osmotic effect between the interior and exterior of the retaining element. This is particularly advantageous, if a common saline solution is used to inflate the retaining element. Likewise, PUR also provides a good resistance with respect to any fluid usually filled into an inflatable retaining element, such as solutions (saline solution), water (with additives), glycerin, water-glycerin emulsion, gases, etc..

The gastrostomy device can be fabricated, so that a portion of the inner layer of the retaining element is coupled to a portion of the tube. This coupling between the inner layer of the retaining element and the tube may be achieved by an adhesive, welding, solvent bonding or otherwise attaching the inner layer of the retaining element to the tube. The tube of the gastrostomy device may also be formed of PUR or at least an outer portion of the tube is at least in sections made of PUR. This provides for an easy coupling of the retaining element to the tube as well as a good adhesion. Optionally, the outer layer of the retaining element may be disposed in such a manner that it covers the coupling portion between the retaining element and the tube. Thus, a protection of the coupling portion can be achieved by the outer layer.

Alternatively, the retaining element can be coupled to a portion of the tube with its outer layer. For example, if the exterior material of the tube has better capabilities to be coupled to the material of the outer layer of the retaining element, this solution provides for better sealing capabilities between the gastrostomy tube and the retaining element, when in an inflated state.

According to a variant of the gastrostomy device, the retaining element can be coupled to the tube at two portions of the tube spaced apart from each other. This provides for the distal end of the tube (the distal end tip of the tube) to be spaced apart from the retaining element. When inserted into the stomach of the patient, such gastrostomy device allows any fluid to be introduced directly into the stomach and prevents the fluid from flowing around the retaining element (sticking to the outer surface of the retaining element) and towards the stoma.

According to a further variant of the gastrostomy device, the retaining element includes at least one tubular extension having a cross-section configured for coaxially sheathing the tube before the at least one tubular extension is coupled (adhered) to the tube. This tubular extension can extend from the inner layer of the retaining element away from an interior space formed by the retaining element. The cross-sectional area of the tubular extension can correspond to a cross-sectional form of the tube. This facilitates inserting the tube into the interior space of the retaining element, so that the tubular extension sheathes the tube.

Furthermore, the retaining element can include two tubular extensions on opposite sides of the retaining element. This allows the tube to pass through the interior space of the retaining element. It further allows the distal end tip of the tube to be spaced apart from the retaining element at least for the same length as the tubular extension facing the distal end tip of the tube.

According to a further optional variant of the retaining element, a connecting portion between the retaining element and the tubular extension(s) has a curved form. This avoids that any fluid (e.g., body fluid or nutrition fed through the gastrostomy tube) accumulates in a (sharp) corner between the tubular extension(s) and the retaining element. Furthermore, at the side of the retaining element that will contact the stomach wall such curved connecting portion further improves a pressure distribution on the stomach wall, and in particular at the stoma formed at the stomach wall.

Alternatively or additionally, the retaining element may have a form where, in a cross-sectional view along a longitudinal axis of the tubular extensions, one or both of the tubular extensions lie closer to a center of the retaining element than parts of the retaining element. In other words, a portion of a tubular extension being closest to the center of the retaining element is tangent to a first plane perpendicular to the longitudinal axis of the tubular extension. A portion of the retaining element adjacent to the tubular extension may be tangent to a second plane perpendicular to the longitudinal axis that is further away from the center of the retaining element than the first plane. Thus, the retaining element forms a bulge lying radially outward from the one or both tubular extension(s).

In case the tubular extension facing the distal end tip of the tube lies closer to the center of the retaining element (i.e. at least partially inside the outer surface of the retaining element), the distal end tip of the tube does not protrude out of the retaining element. Thus, the retaining element forms a support of the gastrostomy device to a side of the stomach that is opposite to the stomach wall where the stoma is formed. This is particularly advantageous, since it significantly reduces a risk that a protruding distal end tip of the tube irritates the opposite side of the stomach.

The outer layer made of polyamide can extend at least partially on the at least one tubular extension. Therefore, the outer layer covers the retaining element as well as the tubular extension(s) thereof. This provides for the same protection of the tubular extension(s) as for the retaining element due to the material of the outer layer. In case of a co-extruded tube, from which the retaining element is formed, the outer layer will fully cover the inner layer at the at least one tubular extension.

According to a variant of the gastrostomy device, the retaining element, in an inflated state, has a substantially ellipsoidal form. The retaining element of ellipsoidal form can be disposed with respect to the tube in such a manner that a plane defined by the widest extension of the retaining element is substantially perpendicular to a longitudinal axis of the tube. Since the tube will pass through the abdominal wall and the stomach wall preferably perpendicularly, the contacting surface between the stomach wall and the such disposed retaining element of ellipsoidal form will be maximized. Therefore, the pressure distribution of the retaining element on the stomach wall is improved.

Additionally or alternatively to the above variants, the retaining element can have a flat portion substantially perpendicular to a longitudinal axis of the tube. Due to the form stability of the retaining element made out of PA it is possible to provide a retaining element that has at least one flat or plane portion. This flat or plane portion is preferably disposed at a portion of the retaining element forming the contacting surfaces with the stomach wall. Thus, the pressure distribution of the retaining element against the stomach wall can be optimized and the retaining capabilities are improved.

The remaining portion of the retaining element can be of a substantially ellipsoidal form. Additionally or alternatively, the retaining element can have a second flat or plane portion opposite to the first flat or plane portion of the retaining element. For instance, two flat or plane portions can extend substantially perpendicular from the tube at two portions of the tube spaced apart from each other. The intermediate portions connecting the two flat or plane portions can have a round shape, for example can have a cross-sectional form corresponding to a portion of a circle or an ellipse.

Furthermore, a surface of the retaining element, when in an empty state, can have an area substantially equal to an area of the surface of the retaining element, when in an inflated state. Due to the form stability of the retaining element the size of the surface of the retaining element does not change between an empty state and an inflated state of the retaining element.

Therefore, the retaining element, when in an empty state, can be folded around the tube and can firmly abut against the outer surface of the tube. The retaining element, when in the empty state, can be folded towards a distal end tip of the tube. It is advantageous if the radial extent of the inflated retaining element (away from the longitudinal center axis of the tube) is so dimensioned that the empty retaining element when folded towards the distal end tip of the tube extends further in a longitudinal direction of the tube than the tube itself. In other words, the folded retaining element covers the distal end tip. This prevents the end of the folded retaining element (contemplated in a longitudinal direction of the tube) to be sharply bend or kinked. It further allows to fold the retaining element next to the distal end tip to an outer dimension (diameter) substantially corresponding to an outer dimension (diameter) of the tube. Thus, insertion of the gastrostomy device into the stomach of the patient is not hindered by the folded retaining element.

According to another variant, the gastrostomy device can further comprise a protection sheath sheathing the retaining element in its empty state, in which state the retaining element is folded around the tube. This protection sheath protects the retaining element during transportation and before insertion into the patient's stomach. The inner dimension of the protection sheath can be sized corresponding to an outer dimension (diameter) of the tube and/or an outer dimension (diameter) of the folded retaining element folded around the tube. The size of the protection sheath is preferably chosen, so that the gastrostomy device can be pushed through the protection sheath easily. Additionally or alternatively, the inner surface of the protection sheath can be made of or can be covered by a material that facilitates gliding of the retaining element and the tube through the protection sheath. The outer layer of the retaining element made of PA facilitates gliding of the gastrostomy device inside the protection sheath. Additionally, the retaining element and/or the outer surface of the tube may be provided with a coating facilitating gliding of the gastrostomy device inside the protection sheath.

For example, when a trocar punctures the abdominal wall and the stomach wall, a further sheath is placed through the abdominal and stomach walls, in the following referred to as an access sheath. When the trocar is removed, the access sheath stays in place. The gastrostomy device can then be inserted through the access sheath. The protection sheath covering the retaining element in its empty state can be coupled to a proximal end of the access sheath. In this state, it is advantageous if the inner surfaces of the protection sheath and of the access sheath align in a longitudinal direction thereof, i.e. in case of tubular protection and access sheaths both have the same inner diameter. The inner size of the protection and access sheaths can be slightly bigger than an outer dimension of the tube or an outer dimension of the folded retaining element around the tube.

In accordance with a variant of the gastrostomy device, the tube can comprise a gastric lumen extending in a longitudinal direction of the tube and a retainer lumen extending in a longitudinal direction of the tube. The retainer lumen can be in fluid communication with an interior space of the retaining element. Furthermore, the gastric lumen can extend to a distal end tip of the tube and is configured to let nutrition and/or medication and/or gas pass through.

For instance, the retainer lumen can be provided with an opening to an exterior of the tube where the retaining element is disposed. In more detail, an opening in the outer tube skin may be arranged at a longitudinal position of the tube fully covered by the retaining element, so that any fluid running through the retainer lumen fills up or empties the interior space of the retaining element. Between the opening in the outer tube skin and the distal end tip of the tube the retainer lumen can be blocked, e.g., by filling up the retainer lumen. Alternatively, the retainer lumen may be squeezed or pressed together in a manner that the lumen is closed. For example, an adhesive or heat can be employed for closing the retainer lumen. Also alternatively, the tube may be formed differently in this section, i.e., without a retainer lumen. Especially if the retainer lumen is fabricated by extrusion, different longitudinal sections of the tube can be formed with different cross-sectional shapes.

According to a further variant, the retainer lumen has a cross-section forming an ellipse, wherein a minor axis of the ellipse aligns with a radially direction of a cross-section of the tube. Thus, the retainer lumen can be fitted into an outer skin of the tube in a space-saving manner. Furthermore, the retainer lumen of such cross-section and arrangement provides for greater curvatures of the cross-section of the gastric lumen, so that the cross-section of the gastric lumen does not have niches. In addition, such retainer lumen facilitates the creation of an opening, so that the retainer lumen and the interior space of the retaining element are in fluid communication with each other. The retainer lumen, where facing the exterior side of the tube, extends wider in a circumferential direction than compared to a circular cross-section or different arrangement of the retainer lumen in the tube. This facilitates the creation of an opening into the retainer lumen.

Furthermore, since the retainer lumen has to transport water, a (saline) solution, glycerin, water-glycerin emulsion, air or a similar gas, the cross-sectional size of the retainer lumen can be smaller than that of the gastric lumen which transports more viscous fluids. For example, the cross-sectional area of the retainer lumen can be between 3% and 10% of a cross-sectional area of the gastric lumen.

In yet another variant the tube can comprise indication elements disposed circumferentially and/or along a longitudinal direction of the tube. In a circumferential direction, the indication elements can be uniformly distributed, such as three indication elements every 120°. At least some of the indication elements can be spaced apart from each other in the longitudinal direction of the tube by a predetermined distance. For example, at least some of the indication elements can be provided at equal intervals, such as 0.5 cm, 1 cm, 1.5cm, or a similar distance providing a graduation of an insertion length of the gastrostomy device.

At least some of such indication elements can be visible from the outside of the tube. For instance, the indication elements can be printed onto an exterior surface of the tube. Alternatively or additionally, at least some of the indication elements can be integrated into a wall of the tube. An exemplary indication element can be integrated into the wall of the tube during fabrication of the tube. For instance, when the tube is fabricated by extrusion, a different material, colored material (same as the remaining extruded material), ink, etc. can be introduced into the tube wall. An indication element fully integrated into the tube wall reduces the risk of a burst of the tube. In particular, if the material of the tube does not fully coat an outer surface of the indication element, a predetermined breaking point may be formed where the material of the tube may break, e.g. due to an internal pressure inside the tube. Likewise, the indication element being of a different material than the tube may uncouple from the material of the tube, thereby damaging the tube.

Furthermore, also alternatively or additionally, each indication element may include a contrast agent. Thus, at least some of the indication elements can form markers. This allows for detecting the indication elements (markers) and, hence, the tube under x-ray or similar technique. Such markers can be provided continuously in a longitudinal direction of the tube. This allows confirming the position of the tube inside the patient's body by corresponding techniques, such as x-ray.

In accordance with another variant, at least a part of the tube is made from polyurethane, PUR. For example, the entire tube can consist of PUR. Alternatively, only certain parts of the tube are made from PUR, such as the part(s) where the retaining element is coupled to the tube. This is particularly advantageous, since the inner layer of the retaining element made from PUR can be coupled easily and in a watertight manner to the tube made from PUR. For example, the retaining element can be coupled to the tube by adhering, solvent bonding or welding it to the tube.

Furthermore, the tube can be covered with a layer of material different from PUR.

This allows provisioning particular properties to the tube, such as better gliding capabilities with respect to other materials (e.g. of an access sheath), a better resistance against body fluids or chemicals used when placing a gastrostomy device, etc.

In accordance with another aspect, polyurethane, PUR, is used for an inner layer of an inflatable retaining element of a gastrostomy device, and polyamide, PA, for an outer layer of the inflatable retaining element.

In accordance with yet another aspect of the present disclosure, a gastrostomy kit comprises a trocar, one or more sheaths (such as a protection sheath and/or an access sheath), and a gastrostomy device including a gastrostomy tube and an inflatable retaining element made of polyurethane, PUR, forming an inner layer and polyamide, PA, forming an outer layer of the retaining element.

It is to be understood that the described aspects and variants do not restrict the present disclosure to the exemplary aspects and variants.

Preferred examples of a gastrostomy device are described in greater detail with reference to the attached schematic drawings in the following, wherein
- Figures 1A and 1B: show a perspective view and a side view of a gastrostomy device, respectively, including a protection sheath sheathing a portion of the gastrostomy device,
- Figure 2: shows a side view of a gastrostomy device including a protection sheath sheathing a portion of the gastrostomy device and a trocar sheathed by an access sheath,
- Figure 3: shows a side view of a gastrostomy device including a protection sheath and an access sheath each sheathing a respective portion of the gastrostomy device,
- Figures 4A to 4D: show a distal end of a gastrostomy device in more detail, and in particular show a retaining element at a distal end of a gastrostomy device sheathed by a protection sheath and an access sheath at different states,
- Figures 5A to 5C: show a sectional side view of respective variants of a retaining element in an inflated state at a distal end of a gastrostomy device,
- Figures 6A and 6B: show exemplary double layer tubes from which a retaining element can be formed,
- Figure 7: shows a sectional side view of a retaining element and increased details thereof,
- Figure 8: shows a side view and a perspective view of a retaining element, and
- Figures 9A and 9B: show a cross-section of gastrostomy tubes of exemplary sizes CH10 and CH14, respectively.

The following detailed description of the schematic drawings focuses on the illustrated implementation variants of a gastrostomy device. The present disclosure is not limited to the above and below described and illustrated gastrostomy devices, but includes combinations of the described variants and implementation details of these gastrostomy devices.

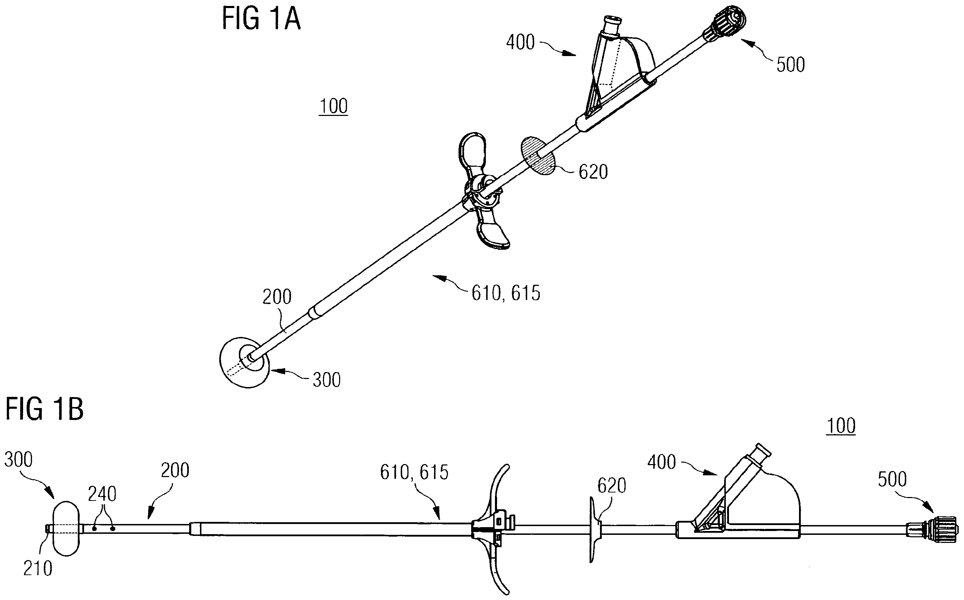

Figures 1A and 1B show a perspective view and a side view of a gastrostomy device 100, respectively. The gastrostomy device 100 includes a gastrostomy tube 200, which forms the main component of the gastrostomy device 100 in a longitudinal direction thereof. At or near the distal end of the gastrostomy tube 200 is an inflatable retaining element 300 coupled to the tube 200. The distal end of the tube 200 and the retaining element 300 will be inserted into a body cavity of a patient, such as the stomach, when the retaining element 300 is in an empty state. Nevertheless, for a better understanding of the overall structure of the gastrostomy device 100 Figures 1A and 1B illustrates the gastrostomy device 100 with an inflated retaining element 300.

In order to facilitate inflation of the retaining element 300, the tube 200 comprises a first lumen, i.e. a retainer lumen, extending in a longitudinal direction of the tube 200. The retainer lumen is in fluid communication with an interior space of the retaining element 300. A connector arrangement 400 is provided at a proximal part of the tube 200. A connector of the connector arrangement 400 is in fluid communication with the retainer lumen and provides for coupling of filling means thereto, so that a fluid can be filled from the filling means through the retainer lumen into the interior space of the retaining element 300. Thereby the retaining element 300 is inflated (filled with the fluid) and achieves a form exemplarily shown in Figures 1A and 1B. The fluid may be water, a saline solution, glycerin, water-glycerin emulsion, air or other gas.

When inflated, the retaining element 300 retains the gastrostomy device 100 in the body cavity of the patient, such as the stomach. A user can pull the gastrostomy device 100, so that the retaining element 300 abuts against an interior wall of the body cavity, such as the stomach wall. For example, the user can pull the gastrostomy device 100 at the connector arrangement 400. Then the gastrostomy device 100 can be secured with an external retaining element 620. The external retaining element 620 is slidably arranged on the tube 200 but withstands a certain pulling or pushing force due to friction between the external retaining element 620 and an outer surface of the tube 200.

In order to facilitate such pulling, the tube 200 includes indication elements 240. Such indication elements 240 can be disposed circumferentially and/or along a longitudinal direction of the tube 200. The indication elements 240 can have an arbitrarily chosen form. For instance, some of the indication elements 240 are continually provided along a longitudinal direction of the tube 200 (not shown in Figure 1A) or can be arranged at equal intervals along a longitudinal direction of the tube 200 as illustrated in Figure 1A. Alternatively, the indication elements 240 can be arranged at logarithmic intervals in the longitudinal direction of the tube 200, where the distances between two adjacent indication elements 240 becomes smaller the closer they are located to the distal end tip 210 or to the retaining element 300. Furthermore, the indication elements 240 can also be provided with numerals, in order to indicate a distance to the distal end tip 210 or a distance to the retaining element 300. For example, the numerals can indicate such distance in centimeters or millimeters.

Furthermore, the tube 200 further includes a second lumen, i.e. a gastric lumen, extending in a longitudinal direction of the tube 200. The gastric lumen extends from a proximal end up to a distal end tip 210 of the tube 200. The gastric lumen is configured to let nutrition and/or medication and/or gas(es) pass through. Therefore, nutrition, medication and/or gas(es) can be led into or removed from the body cavity of the patient, such as the stomach. In order to facilitate supply of nutrition, medication and/or gas(es), a fitting 500 or fitting arrangement is provided at the proximal end of the tube 200.

Figures 1A and 1B further illustrate an exemplary sheath 610, 615 sheathing a portion of the gastrostomy device 100. Such sheath 610, 615 can be employed to form an access sheath into the body cavity, for example, into the stomach through the abdominal wall and stomach wall. Alternatively, such sheath 610, 615 may be a protection sheath for the retaining element 300, when it is in an empty state, for example, for transportation and before and during insertion into the body cavity. In order to remove the sheath 610, 615, it can be teared apart by pulling the two illustrated handles away from each other. The sheath 610, 615 is provided with a predetermined breaking line along the longitudinal direction of the sheath 610, 615.

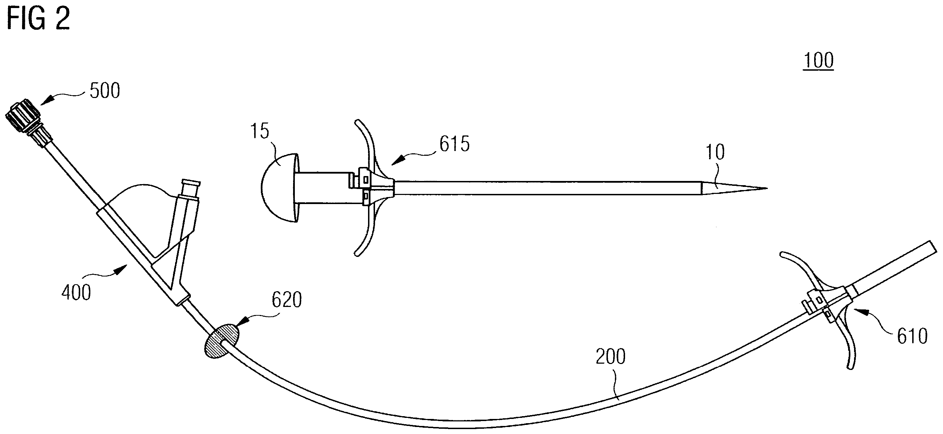

Figure 2 shows a side view of a gastrostomy device 100 and of a trocar 10, each including a sheath 610, 615 sheathing a portion of the gastrostomy device and the trocar, respectively. The illustrated gastrostomy device 100 and trocar 10 can be part of a gastrostomy kit, which is provided "ready for use". The gastrostomy device 100 includes the same components as the gastrostomy device 100 of Figures 1A and 1B, so that the description of such components is omitted for brevity of the present disclosure.

The trocar 10 provided in the gastrostomy kit is inserted into an access sheath 615 and can be locked at a handle at the access sheath 615. The trocar 10 can then be used to puncture an opening through the abdominal wall and stomach wall of the patient by holding the trocar 10 at the knob 15 and pressing the sharp end of the trocar 10 through the abdominal wall and stomach wall. The trocar 10 can be made of stainless steel or PEEK (Polyether ether ketone) with a sharp distal pin end. When inserted, a distal end of the access sheath 615 enters the stomach of the patient. For example, the access sheath 615 can have a size of CH 10 to CH 20 (CH = Charríere, a measuring unit for the outer diameter of tubular devices; 1 CH is approximately 0.33 mm) and may include Teflon to facilitate relative movement of the access sheath 615 and the trocar 10.

This process can be observed with a simple endoscope provided into the stomach of the patient. Since the endoscope is only used to inflate the stomach with air and to provide an image from the inside of the stomach, the smallest available endoscope can be used. For example, a small tube endoscope (having a diameter of only 4.3 mm) can be used, which may be inserted through the nose of the patient. This helps reducing the risk of moving any infectious germs from the throat and/or esophagus of the patient into the stomach and is more comfortable for the patient.

Once inserted, the trocar 10 is removed, while the access sheath 615 maintains in the body of the patient. A valve in the access sheath 615 can close when the trocar 10 is removed, in order to prevent air from escaping from the inflated stomach. For instance, the valve can be disposed in a handle of the access sheath 615. Subsequently, the gastrostomy device 100 can be inserted through the access sheath 615.

As illustrated in Figure 2, a distal end of the gastrostomy tube 200 is covered by a protection sheath 610. The protection sheath 610 can be identically constructed as the access sheath 615. However, the protection sheath 610 can be shorter than the access sheath 615. The protection sheath 610 protects the retaining element 300 provided at the distal end part of the tube 200.

In order to facilitate insertion of the gastrostomy device 100 into the patient's body cavity, the protection sheath 610 can be abutted against a proximal end of the access sheath 615. Thereby the tubular parts of the access and protection sheaths 610, 615 can be brought into alignment, so that both sheaths 610, 615 form a continuous tube. The distal end of the gastrostomy device 100, and in particular the distal end of the tube 200, is pushed through the protection sheath 610 into the access sheath 615 and further into the body cavity of the patient. This insertion can still be observed with the endoscope. By connecting the protection sheath 610 and the access sheath 615 a valve in the access sheath 615 (e.g. in the handle thereof) can be opened. Alternatively, the valve can be opened by the distal end of the tube 200 when pushed through the protection and access sheaths 610, 615.

When brought in place, the retaining element 300 is inflated. This retains the gastrostomy device in the body cavity. Then the access and protection sheaths 610, 615 can be removed from the patient and the gastrostomy device 100 by tearing them apart. Thereafter, the tube 200 can be pulled in a longitudinal direction away from the patient's body abutting the retaining element 300 to the body cavity wall (e.g., stomach wall). Then the gastrostomy device 100 is secured with an external retaining element 620. The external retaining element 620 can be a disk-like element that glides over an exterior surface of the tube 200. When pulling the gastrostomy device 100 away from the patient's body and, at the same time, pushing the external retaining element 620 along the tube 200 towards the patient's body, the abdominal wall and stomach wall are squeezed together and hold in place by both retaining elements 620, 300. In this position, a stoma is formed within two to four weeks.

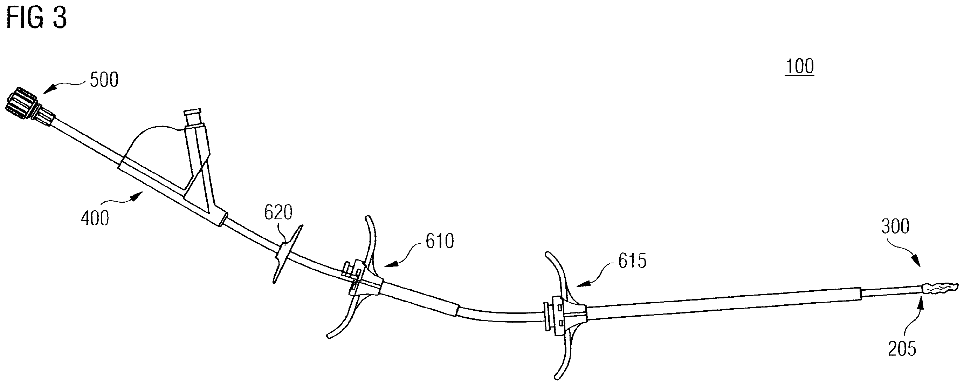

Figure 3 shows a side view of a gastrostomy device 100 including the two sheaths 610, 615 sheathing parts of the gastrostomy tube 200. In particular, Figure 3 illustrates how the distal end 205 of the tube 200 and the retaining element 300 protrude from a distal end of the access sheath 615. This corresponds to the inserting process, where the empty retaining element 300 enters the body cavity.

The protection sheath 610 is illustrated spaced apart from the access sheath 615. However, it is to be understood, that during the insertion process, the protection sheath 610 may abut against the proximal end of the access sheath 615 forming a continuous passage for the gastrostomy tube 200.

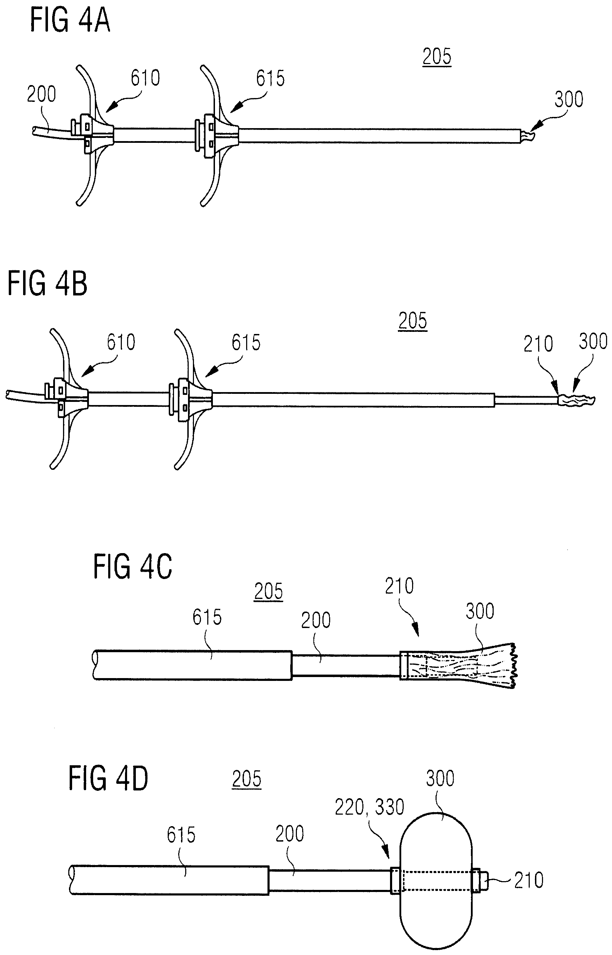

Figures 4A to 4D show an enlarged distal end of a gastrostomy device 100, and in particular a distal end 205 of a gastrostomy tube 200 and a retaining element 300 sheathed by an access sheath 615 and a protection sheath 610 and further show different states of the retaining element. Figure 4A illustrates how the access and protection sheaths 610, 615 form a continuous passage. This drawing further shows that a first portion (distal end portion) of the empty retaining element 300 protrudes from a distal end of the access sheath 615. This will take place while the access sheath 615 is provided through the abdominal wall and stomach wall of the body of the patient.

When pushed further, the entire empty retaining element 300 and the distal end tip 210 of the tube 200 will protrude from the distal end of the access sheath 615, as illustrated in Figure 4B. In its empty state the retaining element 300 can cover the distal end tip 210 of the tube 200. Thus, the empty retaining element 300 forms a distal end of the gastrostomy device 100 during the insertion process. From the enlarged view of the distal end 205 of the tube 200 shown in Figure 4C it is derivable that the retaining element 300 takes up space adjacent to the distal end 210 of the tube 200 in a longitudinal direction of the tube 200. This facilitates folding the retaining element 300 and providing it within the protection sheath 610. Since the distal end of the folded retaining element 300 can use the entire cross-sectional area of the protection sheath 610, kinks or other sharp folding edges of the retaining element 300 are avoided.

A surface of the retaining element 300 in its empty state has an area substantially equal to an area of the surface of the retaining element 300 when in its inflated state. In other words, the material of the retaining element 300 has almost no prolongation when being inflated. Thus, the empty retaining element 300 when being stowed requires more volume than, for example, an elastically extendable retaining element, such as the conventional retaining element made from elastic silicone.

Figure 4D shows the distal end 205 of the tube 200, where the retaining element 300 is inflated. As illustrated in Figure 4D, a portion 330 of the inner layer of the retaining element 300 is coupled to a portion 220 of the tube 200. Furthermore, the retaining element 300 is coupled to the tube 200 at two portions of the tube 200 spaced apart from each other, although only one portion 220 is visible in the drawing. The distal end 210 of the tube 200 protrudes from the retaining element 300. This facilitates the effusing of nutrition, medication and/or gas(es) from the distal end 210 of the tube 200.

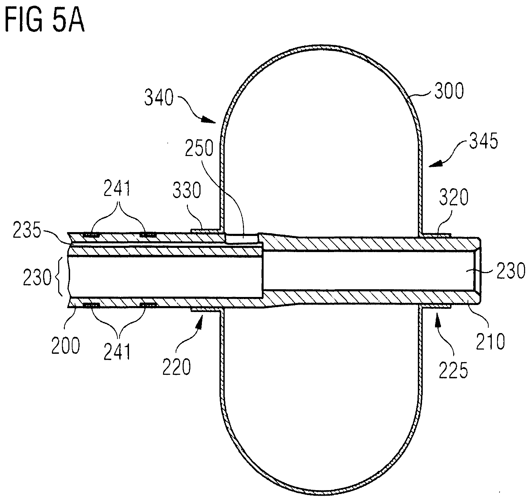

Figure 5A shows a cross-sectional view of a retaining element 300 in an inflated state at a distal end 205 of a gastrostomy tube 200. The retaining element 300 includes at least one tubular extension 320, 330 having a cross-section configured for coaxially surrounding the tube 200. In particular, a first tubular extension 320 surrounds a first portion 225 of the tube 200 and a second tubular extension 330 surrounds a second portion 220 of the tube 200.

The tube 200 includes a gastric lumen 230 and a retainer lumen 235 both extending in a longitudinal direction of the tube 200. Between the first portion 225 and the second portion 220 of the tube 200 an opening 250 in an outer skin of the tube 200 may be provided. This opening 250 provides for a fluid communication between the retainer lumen 235 and an interior space of the retaining element 300. Any fluid provided into the retainer lumen 235 can flow into the interior space of the retaining element 300, thereby inflating the retaining element 300.

The retaining element 300 comprises an inner layer made of polyurethane (PUR) and an outer layer made of polyamide (PA). The tubular extensions 320, 330 can be formed at least from the inner PUR-layer. A watertight connection is provided between the tubular extensions 320, 330 and the first and second portions 225, 220 of the tube 200, respectively. For example, the tubular extensions 320, 330 can be welded, adhered, shrink fitted, etc. onto the first and second portions 225, 220 of the tube 200, respectively. According to an example, at least portions 220, 225 of the tube 200 can be made of polyurethane (PUR), so that a solvent or heat bonding between the tubular extension 320, 330 (made also of PUR) with the respective portion 220, 225 of the tube 200 can be achieved easily. It is to be understood that the entire tube 200 can be made of PUR. The retaining element 300 can include an outer layer made of polyamide (PA). The outer PA-layer can extend at least partially on the at least one tubular extension 320, 330. In addition, the PA-layer can cover the entire tubular extensions 320, 330 as well as a part or all of an exterior surface of the tube 200. For instance, when the retaining element 300 is fixed to the tube 200, most parts of the gastrostomy device 100 can be dipped into a solution coating the retaining element 300 and the tube 200 with a PA-layer. Alternatively, the retaining element 300 is formed from a double layer tube consisting of PUR and PA layers and the tube 200 is also a double layer tube consisting of an inner PUR layer and an outer PA layer. In this case, the PA layer can be removed from the tube 200 at the two portions where the tubular extensions 320, 330 will be adhered, so that the PUR-layers of the tube 200 and the tubular extensions 320, 330 come into contact with each other.

Figure 5A further shows that the tube 200 can have different cross-sections along a longitudinal axis of the tube 200. For example, with the opening 250 into the retainer lumen 235 the retainer lumen 235 can end. Therefore, the tube 200 may be formed between the opening 250 and the distal end tip 210 with a cross-section having only the gastric lumen 230. This allows a smaller outer diameter of the distal end of the tube 200, creating more space in particular in an area where the empty retaining element is disposed when folded.

Furthermore, the tube 200 may be provided with indication elements 240, such as symbols 241 or tick marks of a scale. Such symbols 241 are provided on a portion of the tube 200 facing towards the proximal end of the tube, i.e. a portion that will lie outside of the body of the patient when the gastrostomy device 100 is placed in the patient's body cavity. The indication elements 240 may be arranged circumferentially and/or in a longitudinal direction of the tube 200 at any desired distance. In a circumferential direction, the indication elements 240 can be uniformly distributed, such as three indication elements every 120° (not shown in Figure 5A). The symbols 241 can be spaced apart from each other in the longitudinal direction of the tube 200 by a predetermined distance. For example, symbols 241 can be provided at equal intervals, such as 0.5 cm, 1 cm, 1.5cm, or any other distance required to form a scale indicating a distance to the retaining element 300 and, in particular, a distance to the surface 340 of the retaining element 300 abutting the stomach wall. The symbols 241 may surround the tube 200 entirely (forming rings on a surface of the tube 200 as illustrated in Figure 5A) or may be subdivided in a circumferential direction. The indication elements 240 can be printed onto the surface of the tube 200 or may be integrated into the tube wall of tube 200.

As can be derived from Figure 5A, the retaining element 300, in an inflated state, can have a substantially ellipsoidal form. It is to be understood that the retaining element 300 does not need to have a perfectly symmetrical ellipsoidal form. For instance, the retaining element 300 may comprise one or more flat or plane surfaces 340, 345. It is particularly advantageous if a surface that will abut against the stomach wall of the patient has a flat or plane portion 340, since this provides good retaining properties. The retaining properties can be enhanced, if the flat or plane portion 340 is disposed substantially perpendicular to a longitudinal axis of the tube 200.

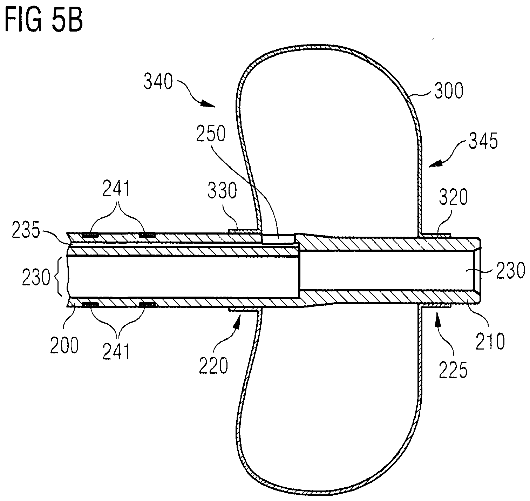

Alternatively, as illustrated in Figure 5B, the flat or plane surface 340 of Figure 5A may be bent inwardly, i.e. towards a center of the retaining element 300. This can be achieved by moving the proximal tubular extension 330 closer to the distal end of the tube 200 before adhering the proximal tubular extension 330 to the tube 200. Alternatively or additionally, the retaining element 300 may be fabricated as having a bulge that is maintained due to the form durability of the PUR- and PA-layers of the retaining element 300, where the proximal tubular extension 330 lies closer to a center of the retaining element 300. This provides additional flexibility for the gastrostomy device 100 when the retaining element 300 abuts against the wall of the body cavity, such as the stomach wall, around the stoma. Thus, the wall of the body cavity is less irritated by the proximal tubular extension 330 and/or the surface 340 of the retaining element 300.

Additionally or alternatively, the retaining element 300 can be provided with a bulgy form on the opposite (distal) side, i.e. at surface 345. Likewise, the distal tubular extension 320 may be adhered to the tube 200 resulting in the bulgy form of the retaining element 300 and/or the retaining element 300 may be fabricated with such bulgy form.

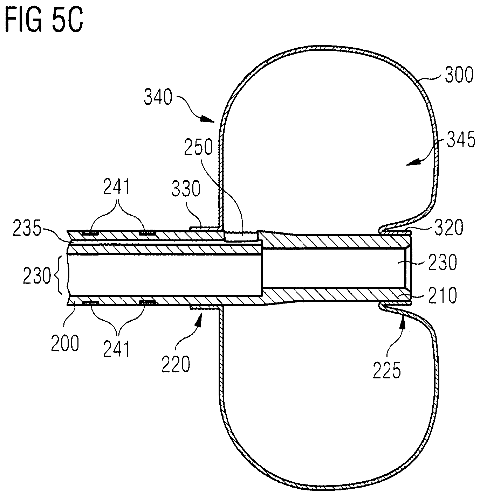

Also additionally or alternatively, the retaining element 300 can be provided with a flat or plane surface 345 on its distal side and the distal tubular extension 320 is brought closer to the proximal end of the tube 200 before adhering the distal tubular extension 320 to the tube 200. For instance, the flat or plane surface 345 can protrude further to a distal side of the gastrostomy device 100 than the distal end tip 210 of the tube 200. Such form of the retaining element 300 is illustrated in Figure 5C. This allows providing the distal end tip 210 closer to the center of the retaining element 300. This form of the retaining element 300 and, in particular, the surface 345 of the inflated retaining element 300 then facilitates keeping the distal end tip 210 away from a corresponding wall of the body cavity (otherwise pricking into the wall of the body cavity), such as the stomach wall. Thus, the wall of the body cavity on the distal side of the retaining element 300 will not be irritated (or less irritated) by the tube 200 (particularly its distal end tip 210) and/or the surface 345 of the retaining element 300.

It is to be understood that distal side surface 345 of the retaining element 300 is not limited to the form illustrated in Figure 5C. The flat or plane surface 345 can instead be flush with the distal end tip 210 of the tube 200. Furthermore, it is also understood that the proximal side (surface 340) of the retaining element 300 can be formed corresponding to the distal side (surface 345) of the retaining element 300.

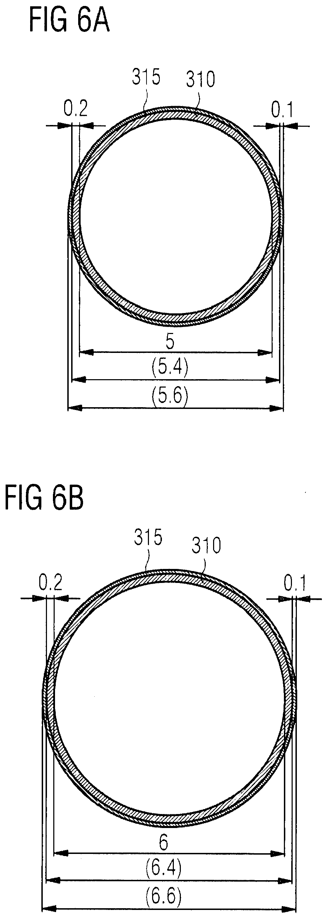

In order to fabricate any of these retaining elements 300, a double layer tube may be formed as illustrated in Figures 6A and 6B. For example, a double layer tube can be fabricated by coextrusion of PUR forming an inner layer 310 of the tube and PA forming an outer layer 315 of the tube. Figure 6A shows an exemplary tube having an outer diameter of approximately 5.6 mm and an inner diameter of approximately 5 mm. The thickness of the inner PUR-layer 310 can be between 0.1 mm and 0.4 mm, preferably 0.2 mm, and the thickness of the outer PA-layer 315 can be between 0.05 mm to 0.2 mm, preferably 0.1 mm. Such tube can be used to form a retaining element 300 that fits onto (can be coupled to) a tube 200 of size CH 10.

According to another example illustrated in Figure 6B, the double layer tube may have an outer diameter of approximately 6.6 mm and an inner diameter of approximately 6 mm. The thickness of the inner PUR-layer 310 can be between 0.1 mm and 0.4 mm, preferably 0.2 mm, and the thickness of the outer PA-layer 315 can be between 0.05 mm to 0.2 mm, preferably 0.1 mm. Such tube can be used to form a retaining element 300 that fits onto (can be coupled to) a tube 200 of size CH 14.

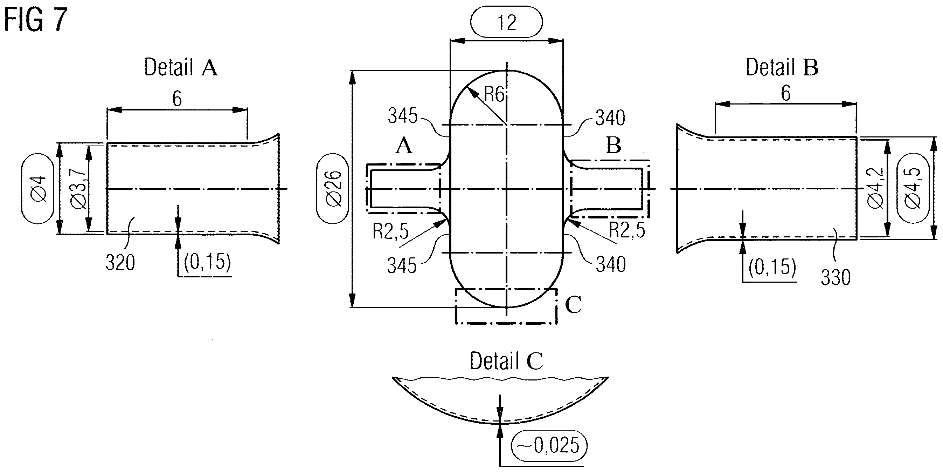

Such double layer tube is then expanded or widened to take the form of the retaining element 300, as shown in Figure 7. Optionally, the double-layered tube can be elongated or stretch formed before widening. After (elongating and) curing , the retaining element 300 can be taken out of a mold or tool. Any remaining parts of the double layer tube at one or both ends of the retaining element 300 can be cut to a desired length, thereby providing the proximal tubular extension 330 and/or the distal tubular extension 320.

During the elongating the diameter of the double-layered tube decreases. By elongating adjacent portions of the double-layered tube to a different extent, varying diameters of the double-layer tube can be achieved. This allows forming tubular extensions 320, 330 that fit onto different portions of tube 200, such as portions 220, 225 (Figure 5A) having different diameters. It is to be understood that both tubular extensions 320, 330 can have the same inner diameter, so that they fit onto a tube 200 having a continuous outer diameter. Furthermore, elongating the double-layered tube allows fabricating retaining elements 300 for tubes 200 of different sizes, e.g. CH 8 to CH 12, from the same double-layered tube, such as the double-layered tube illustrated in Figure 6A. Likewise, the double-layered tube illustrated in Figure 6B may be used to fabricate retaining elements 300 for tubes 200 of sizes between CH 12 and CH 20. It is to be understood that a specific double-layered tube (having a specific diameter) can be employed only for a retaining element 300 for a certain tube size.

Only as an example, a first tubular extension 320 of the retaining element 300 may have an outer diameter between 3.9 mm and 4.3 mm, preferably 4 mm, , an inner diameter of approximately 3.7 mm and a wall thickness of approximately 0.15 mm. Similarly, a second tubular extension 330 may have an outer diameter between 4.4 mm and 5 mm, preferably 4.5 mm, an inner diameter of approximately 4.2 mm and a wall thickness of approximately 0.15 mm. As can be derived from these dimensions, the first tubular extension 320 may have a smaller inner (and outer) diameter than the second tubular extension 330, in order to fit onto a smaller portion of the tube 200 at a distal end 205 thereof (see Figure 5).

Furthermore, the retaining element 300 may have a substantially elliptical cross-section, a bulgy cross-section or other partially round form. For instance, the outer ends (in a radially direction) of the retaining element may be curved, for example having a radius between 2.5 mm and 10 mm, preferably 6 mm, so that the retaining element 300 is approximately 5 mm to 20 mm deep, preferably 8 mm to 15 mm deep, and most preferably 12 mm deep (in a longitudinal direction of the tubular extensions 320, 330). The maximum radial extend of the retaining element 300 can be between 20 mm and 35 mm, preferably between 22 mm and 30 mm, and most preferably ca. 26 mm. Since the double layer tube is widened to form the retaining element 300, the wall thickness varies from the tubular extensions 320, 332 the radially maximal spaced portion of the retaining element 300. At the radially maximal spaced portion of the retaining element 300 (cf. Detail C in Figure 7) the wall thickness can become approximately 0.025 mm thin.

As can be further derived from Figure 7, the tubular extensions 320, 330 can migrate to the retaining element 300 with a curvature, for example a radius between 2 mm and 3 mm, preferably 2.5 mm. Alternatively, the transition between tubular extensions 320, 330 and the retaining element 300 may be substantially perpendicular. Between this transitioning portion and the curved radially maximal spaced portion (outer end) of the retaining element 300 may be a flat or plane surface 340, 345. Flat or plane surface 340 is configured for abutting against a stomach wall of the patient. Alternatively, the surface 340 may have a rather bulgy form (Figure 5B). Likewise, surface 345 can be a flat or plane surface or can have a rather bulgy form (Figure 5C).

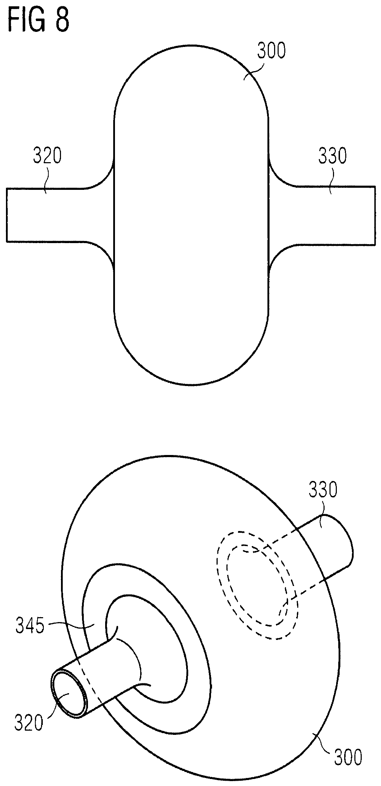

Figure 8 schematically shows a side view and a perspective view of a retaining element 300 thus formed. Only one flat or plane surface 345 of the retaining element 300 is visible. It is to be understood that the retaining element can also be fabricated without a flat or plane surface.

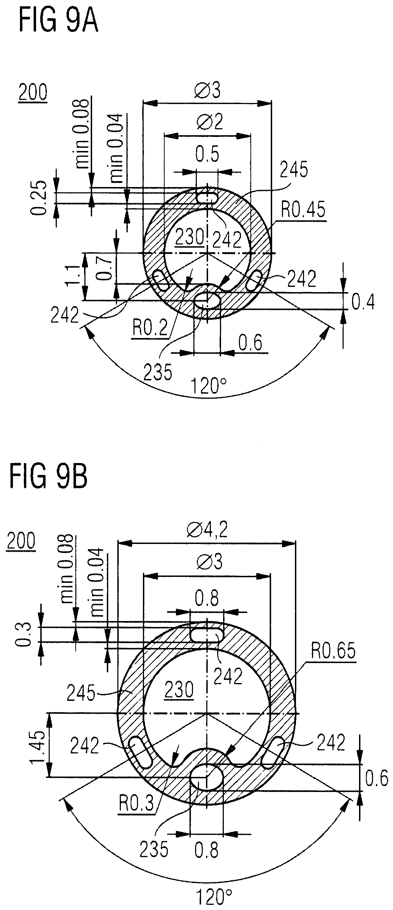

Figures 9A and 9B show cross-sections of gastrostomy tubes 200 of sizes CH10 and CH14, respectively. According to a first example shown in Figure 9A, an outer diameter of the tube 200 can be approximately 3 mm and an inner diameter can be approximately 2 mm.

The tube wall 245 thus formed creates a gastric lumen 230. Within the tube wall 245 can be a retainer lumen 235. Both lumens 230, 235 extend in a longitudinal direction of the tube 200, wherein the gastric lumen 230 extends to a distal end tip 210 of the tube 200 and the retainer lumen 235 is in fluid communication with an interior space of the retaining element 300. Such fluid communication can be achieved by an opening (not shown in Figure 9A) through the tube wall 245 into the retainer lumen 235 (from an exterior side of the tube 200). The retainer lumen 235 can have a cross-section forming an ellipse. Such ellipse may have a minor axis that aligns with a radially direction of a cross-section of the tube 200, as shown in Figure 9A. The minor axis of the ellipse may have a length of approximately 0.4 mm, and a major axis of the ellipse may have a length of approximately 0.6 mm. The center of the ellipse may be about 1.1 mm from the center of the cross-section of the tube 200.

Due to the retainer lumen 235 the cross-section of the gastric lumen 230 has an indent. This indent may be formed by two curved sections moving towards the center of the tube 200, each having a radius of about 0.2 mm and an intermediate section with an opposite curvature with a radius of approximately 0.45 mm. This intermediate section can be substantially parallel to the corresponding part of the ellipse of the retainer lumen 235. Therefore, the indent into the gastric lumen 230, i.e. the interior surface of the tube wall 245 at the most indenting position, may be spaced apart from the center of the tube 200 by approximately 0.7 mm.

Furthermore, integrated into the tube wall 245 may be indication elements 240. For example, Figure 9A depicts indication elements 240 in the form of markers 242, which are arranged in a circumferential direction. The markers 242 can be uniformly distributed, such as three markers 242 every 120°. The markers 242 can have an approximately rectangular or elliptical cross-section having dimensions of approximately 0.5 mm and 0.25 mm. The distance between a marker 242 and an exterior surface of the tube wall 245 may be about 0.08 mm, while a distance between the marker 242 and an interior surface of the tube wall 245 (the surface of the gastric lumen 230) may be about 0.04 mm. The markers 242 may extend over the entire length of the tube 200 (in a longitudinal direction of the tube 200).

According to a second example shown in Figure 9B, an outer diameter of the (CH14) tube 200 can be approximately 4.2 mm and an inner diameter of approximately 3 mm.

The tube wall 245 thus formed creates a gastric lumen 230. Within the tube wall 245 can be a retainer lumen 235. The retainer lumen 235 can have a cross-section forming an ellipse. Such ellipse may have a minor axis that aligns with a radially direction of a cross-section of the tube 200, as shown in Figure 9B. The minor axis of the ellipse may have a length of approximately 0.6 mm , and a major axis of the ellipse may have a length of approximately 0.8 mm. The center of the ellipse may be about 1.45 mm from the center of the cross-section of the tube 200.

Due to the retainer lumen 235 the cross-section of the gastric lumen 230 has an indent. This indent may be formed by two curved sections moving towards the center of the tube 200, each having a radius of about 0.3 mm and an intermediate section with an opposite curvature of a radius of approximately 0.65 mm being substantially parallel to the corresponding part of the ellipse of the retainer lumen 235. Therefore, the indent into the gastric lumen 230 may be closer to the center of the tube 200 than the remaining interior surface of the tube wall 245.

Furthermore, integrated into the tube wall 245 may be indication elements 240. For example, Figure 9B depicts indication elements 240 in the form of markers 242 may be arranged in a circumferential direction. The markers 242 can be uniformly distributed, such as three markers 242 every 120°. The markers 242 can have an approximately rectangular or elliptical cross-section having dimensions of approximately 0.8 mm and 0.3 mm. The distance between a marker 242 and an exterior surface of the tube wall 245 may be about 0.08 mm, while a distance between the marker 242 and an interior surface of the tube wall 245 (the surface of the gastric lumen 230) may be about 0.04 mm. The markers 242 may extend over the entire length of the tube 200 (in a longitudinal direction of the tube 200).

Alternatively or additionally to markers 242 integrated into the tube wall 245, symbols 241 (not shown in Figures 6A and 6B) can also be printed onto an exterior surface of the tube wall 245.

## Claims

1. A gastrostomy device (100), comprising:
a gastrostomy tube (200); and
an inflatable retaining element (300) coupled to the tube (200) at or near a distal end (205) of the tube (200),
**characterized in that**
the retaining element (300) comprises an inner layer (310) made of polyurethane, PUR, and an outer layer (315) made of polyamide, PA.

2. The gastrostomy device (100) according to claim 1, wherein a portion (320, 330) of the inner layer of the retaining element (300) is coupled to a portion (220, 225) of the tube (200).

3. The gastrostomy device (100) according to claim 1 or 2, wherein the retaining element (300) is coupled to the tube (200) at two portions (220, 225) of the tube (200) spaced apart from each other.

4. The gastrostomy device (100) according to one of claims 1 to 3, wherein the retaining element (300) includes at least one tubular extension (320, 330) having a cross-section configured for coaxially sheathing the tube (200).

5. The gastrostomy device (100) according to claim 4, wherein the outer layer (315) made of polyamide extends at least partially on the at least one tubular extension (320, 330).

6. The gastrostomy device (100) according to one of claims 1 to 5, wherein the retaining element (300), in an inflated state, has a substantially ellipsoidal form.

7. The gastrostomy device (100) according to one of claims 1 to 6, wherein the retaining element (300) has a flat portion (340) substantially perpendicular to a longitudinal axis of the tube (200).

8. The gastrostomy device (100) according to one of claims 1 to 7, wherein a surface of the retaining element (300), when in an empty state, has an area substantially equal to an area of the surface of the retaining element (300), when in an inflated state.

9. The gastrostomy device (100) according to one of claims 1 to 8, wherein the gastrostomy device (100) further comprises:
a protection sheath (610) sheathing the retaining element (300) in its empty state, wherein the retaining element (300) in its empty state is folded around the tube (200).

10. The gastrostomy device (100) according to one of claims 1 to 9, wherein the tube (200) comprises a gastric lumen (230) extending in a longitudinal direction of the tube (200) and a retainer lumen (235) extending in a longitudinal direction of the tube (200), wherein the retainer lumen (235) is in fluid communication with an interior space of the retaining element (300), and wherein the gastric lumen (230) extends to a distal end tip (210) of the tube (200) and is configured to let nutrition and/or medication and/or a gas pass through.

11. The gastrostomy device (100) according to claim 10, wherein the retainer lumen (235) has a cross-section forming an ellipse, wherein a minor axis of the ellipse aligns with a radially direction of a cross-section of the tube (200).

12. The gastrostomy device (100) according to one of claims 1 to 11, wherein the tube (200) comprises indication elements (240) disposed circumferentially and/or along a longitudinal direction of the tube (200).

13. The gastrostomy device (100) according to claim 12, wherein at least some of the indication elements (240) are visible from the outside of the tube (200), and/or wherein at least some of the indication elements (240) are integrated into a wall (245) of the tube (200), and/or wherein at least some of the indication elements (240) include a contrast agent.

14. The gastrostomy device (100) according to one of claims 1 to 13, wherein at least a part of the tube (200) is made of polyurethane, PUR.

## Patentansprüche

1. Gastrostomievorrichtung (100), umfassend:
einen Gastrostomieschlauch (200); und
ein aufblasbares Rückhalteelement (300), das mit dem Schlauch (200) an oder in der Nähe eines distalen Endes (205) des Schlauchs (200) verbunden ist,
**dadurch gekennzeichnet, dass**
das Rückhalteelement (300) eine Innenschicht (310) aus Polyurethan, PUR, und eine Außenschicht (315) aus Polyamid, PA, umfasst.

2. Gastrostomievorrichtung (100) gemäß Anspruch 1, wobei ein Abschnitt (320, 330) der Innenschicht des Rückhalteelements (300) mit einem Abschnitt (220, 225) des Schlauchs (200) verbunden ist.

3. Gastrostomievorrichtung (100) gemäß Anspruch 1 oder 2, wobei das Rückhalteelement (300) an zwei voneinander beabstandeten Abschnitten (220, 225) des Schlauchs (200) mit dem Schlauch (200) verbunden ist.

4. Gastrostomievorrichtung (100) gemäß einem der Ansprüche 1 bis 3, wobei das Rückhalteelement (300) mindestens einen schlauchförmigen Fortsatz (320, 330) mit einem Querschnitt enthält, der dazu eingerichtet ist, den Schlauch (200) koaxial zu umhüllen.

5. Gastrostomievorrichtung (100) gemäß Anspruch 4, wobei sich die Außenschicht (315) aus Polyamid zumindest teilweise auf dem mindestens einen schlauchförmigen Fortsatz (320, 330) erstreckt.

6. Gastrostomievorrichtung (100) gemäß einem der Ansprüche 1 bis 5, wobei das Rückhalteelement (300) im aufgeblasenen Zustand eine im Wesentlichen ellipsoide Form aufweist.

7. Gastrostomievorrichtung (100) gemäß einem der Ansprüche 1 bis 6, wobei das Rückhalteelement (300) einen flachen Abschnitt (340) aufweist, der im Wesentlichen senkrecht zu einer Längsachse des Schlauchs (200) verläuft.

8. Gastrostomievorrichtung (100) gemäß einem der Ansprüche 1 bis 7, wobei eine Oberfläche des Rückhalteelements (300) in einem leeren Zustand eine Fläche aufweist, die im Wesentlichen gleich einer Fläche der Oberfläche des Rückhalteelements (300) in einem aufgeblasenen Zustand ist.

9. Gastrostomievorrichtung (100) gemäß einem der Ansprüche 1 bis 8, wobei die Gastrostomievorrichtung (100) ferner umfasst:
eine Schutzhülle (610), die das Rückhalteelement (300) in seinem leeren Zustand umhüllt, wobei das Rückhalteelement (300) in seinem leeren Zustand um den Schlauch (200) herum gefaltet ist.

10. Gastrostomievorrichtung (100) gemäß einem der Ansprüche 1 bis 9, wobei der Schlauch (200) ein Magenlumen (230), das sich in einer Längsrichtung des Schlauchs (200) erstreckt, und ein Rückhaltelumen (235), das sich in einer Längsrichtung des Schlauchs (200) erstreckt, umfasst, wobei das Rückhaltelumen (235) in Fluidverbindung mit einem Innenraum des Rückhalteelements (300) steht, und wobei sich das Magenlumen (230) zu einer distalen Endspitze (210) des Schlauchs (200) erstreckt und dazu eingerichtet ist, Nahrung und/oder Medikamente und/oder ein Gas durchzulassen.

11. Gastrostomievorrichtung (100) gemäß Anspruch 10, wobei das Rückhaltelumen (235) einen Querschnitt aufweist, der eine Ellipse bildet, wobei eine Nebenachse der Ellipse mit einer radialen Richtung eines Querschnitts des Schlauchs (200) fluchtet.

12. Gastrostomievorrichtung (100) gemäß einem der Ansprüche 1 bis 11, wobei der Schlauch (200) Indikationselemente (240) umfasst, die in Umfangsrichtung und/oder entlang einer Längsrichtung des Schlauchs (200) angeordnet sind.

13. Gastrostomievorrichtung (100) nach Anspruch 12, wobei zumindest manche der Indikationselemente (240) von der Außenseite des Schlauchs (200) sichtbar sind, und/oder wobei zumindest manche der Indikationselemente (240) in eine Wand (245) des Schlauchs (200) integriert sind, und/oder wobei zumindest manche der Indikationselemente (240) ein Kontrastmittel enthalten.

14. Gastrostomievorrichtung (100) gemäß einem der Ansprüche 1 bis 13, wobei zumindest ein Teil des Schlauchs (200) aus Polyurethan, PUR, besteht.

## Revendications

1. Dispositif de gastrostomie (100), comprenant:
un tube de gastrotomie (200); et
un élément de retenue gonflable (300) accouplé au tube (200) au niveau d'une extrémité distale (205) du tube (200) ou proche de cette dernière
**caractérisé en ce que**
l'élément de retenue (300) comprend une couche interne (310) constituée de polyuréthanne, PUR, et une couche externe (315) constituée de polyamide, PA.

2. Dispositif de gastrostomie (100) selon la revendication 1, dans lequel une partie (320, 330) de la couche interne de l'élément de retenue (330) est accouplée à une partie (220, 225) du tube (200).

3. Dispositif de gastrostomie (100) selon la revendication 1 ou 2, dans lequel l'élément de retenue (300) est accouplé au tube (200) au niveau de deux parties (220, 225) du tube (200) espacées l'une de l'autre.

4. Dispositif de gastrostomie (100) selon l'une quelconque des revendications 1 à 3, dans lequel l'élément de retenue (300) comprend au moins une extension tubulaire (320, 330) ayant une section transversale conçue pour protéger coaxialement le tube (200).

5. Dispositif de gastrostomie (100) selon la revendication 4, dans lequel la couche externe (315) constituée de polyamide s'étend au moins partiellement sur l'au moins une extension tubulaire (320, 330).

6. Dispositif de gastrostomie (100) selon l'une quelconque des revendications 1 à 5, dans lequel l'élément de retenue (300), lorsqu'il est gonflé, a une forme sensiblement ellipsoïdale.

7. Dispositif de gastrostomie (100) selon l'une quelconque des revendications 1 à 6, dans lequel l'élément de retenue (300) a une partie plate (340) sensiblement perpendiculaire à un axe longitudinal du tube (200).

8. Dispositif de gastrostomie (100) selon l'une quelconque des revendications 1 à 7, dans lequel une surface de l'élément de retenue (300), lorsqu'il est vide, a une zone sensiblement égale à une zone de la surface de l'élément de retenue (300), lorsqu'il est gonflé.

9. Dispositif de gastrostomie (100) selon l'une quelconque des revendications 1 à 8, dans lequel le dispositif de gastrotomie (100) comprend en outre:
une gaine de protection (610) protégeant l'élément de retenue (300) lorsqu'il est vide, dans lequel l'élément de retenue (300), lorsqu'il est vide, est plié autour du tube (200).

10. Dispositif de gastrostomie (100) selon l'une quelconque des revendications 1 à 9, dans lequel le tube (200) comprend une lumière gastrique (230) s'étendant dans une direction longitudinale du tube (200) et une lumière de retenue (235) s'étendant dans une direction longitudinale du tube (200), dans lequel la lumière de retenue (235) est en communication fluidique avec un espace intérieur de l'élément de retenue (300), et dans lequel la lumière gastrique (230) s'étend vers une pointe d'extrémité distale (210) du tube (200) et est conçue pour laisser passer les aliments et/ou des médicaments et/ou du gaz.

11. Dispositif de gastrostomie (100) selon la revendication 10, dans lequel la lumière de retenue (235) a une section transversale formant une ellipse, dans lequel un axe mineur de l'ellipse s'aligne avec une direction radiale d'une section transversale du tube (200).

12. Dispositif de gastrostomie (100) selon l'une quelconque des revendications 1 à 11, dans lequel le tube (200) comprend des éléments d'indication (240) disposés circonférentiellement et/ou le long d'une direction longitudinale du tube (200).

13. Dispositif de gastrostomie (100) selon la revendication 12, dans lequel au moins certains des éléments d'indication (240) sont visibles depuis l'extérieur du tube (200), et/ou dans lequel au moins certains éléments d'indication (240) sont intégrés dans une paroi (245) du tube (200), et/ou dans lequel au moins certains des éléments d'indication (240) comprennent un agent de contraste.

14. Dispositif de gastrostomie (100) selon l'une quelconque des revendications 1 à 13, dans lequel au moins une partie du tube (200) est constituée de polyuréthanne, PUR.
